# EUROPEAN PATENT APPLICATION

(11) **EP 4 786 840 A1**
(43) Date of publication of application: **05.08.2026**
(21) Application number: 25154983.8
(22) Date of filing: 30.01.2025
(51) Int. Cl.: F16M 11/18, A61B 50/13, A61B 90/25, A61B 90/50, F16M 11/42, G02B 7/00, G02B 21/00, G02B 21/36, A61B 90/00

(54) **MOUNTING DEVICE, DISPLAY SYSTEM, MOVABLE CART, OPTICAL IMAGING SYSTEM, OPERATING SYSTEM, METHOD AND COMPUTER PROGRAM**

(71) Applicant: Leica Instruments (Singapore) Pte Ltd, Singapore 618299 (SG)
(72) Inventor: JUNG, David, 618299 Singapore (SG); KOK, Alvin, 618299 Singapore (SG); TAY, Kean Peng, 608924 Singapore (SG); LECK, Kheng Swee, 618299 Singapore (SG); CHARLES, Romy, 608924 Singapore (SG); TAY, Wee Chin, 608924 Singapore (SG); SEE, Andy, 608924 Singapore (SG)
(74) Representative: 2SPL Patentanwälte PartG mbB

(57) **Abstract**

A mounting device (100) is provided which is suitable for arranging a display (110) above an operating table (120), the mounting device further comprising a movable cart (130) suitable for extending transversely under the operating table and an arm (140) suitable for extending next to the operating table, at least one input interface (410) configured to receive data indicating a target position or movement direction of the display relative to a microscope or relative to a user of the microscope, an actuator (420) and control circuitry (430) configured to control the actuator to move at least a display mounting structure of the mounting device for adjusting a position of the display based on the data.

## Description

### Technical field

Examples relate to a mounting device, a display system, a movable cart, an optical imaging system, an operating system, a method and a computer program.

### Background

Efficient use of space and ergonomic design in operating rooms (ORs) can be essential for the success of 3D heads-up surgeries. Conventional setups involve large 55-inch monitors mounted on bulky carts, which occupy valuable floor space and exacerbate the spatial challenges of already crowded ORs. These monitors are often placed at the far end or off-center relative to the patient's bed, forcing surgeons to adopt awkward postures to maintain proper visibility during lengthy procedures. There is a need to optimize space utilization and enhance ergonomic comfort for surgeons to improve the overall surgical workflow and outcomes.

### Summary

This need is met by the independent claims stated herein.

Examples provide a mounting device suitable for arranging a display above an operating table. The mounting device may be a tool, structure, or apparatus configured to hold, support, or attach a display (monitor) to a specific surface or position. Conventional monitor stands used in surgeries may typically be positioned beside the patient, consuming valuable OR space. In contrast, the proposed mounting device may allow the display to be positioned above the patient, reducing or even eliminating its footprint in the OR. Additionally, this setup may bring the display closer to the surgeon during procedures, enhancing visibility and improving ergonomic comfort. As a result, a smaller display (e.g., 27 or 32 inch) can be utilized while maintaining comparable visibility.

The mounting device may comprise, e.g., one or more arms or brackets that hold the display on a wall or the ceiling of the OR and enable arrangement above the operating table. Another option may be an arrangement of the mounting device at an edge of the operating table, e.g., a table-integrated system designed to hold the monitor overhead. Alternatively, the mounting device may comprise a tripod, a stand or a (movable) cart as free-standing device in the OR.

In the case of a ceiling-mounted mechanism, a ceiling bracket may be used to securely mount the display to the OR ceiling, e.g., with a telescoping arm which additionally allows precise placement and height adjustment. The display may directly hang over the patient's bed. Optionally, an articulating arm mechanism may be used for full adjustment capabilities (height, angle, and tilt), allowing the surgeon to position the monitor for optimal visibility and ergonomics. The adjustable arm may permit multiple adjustments: height, angle, tilt, and horizontal positioning. The ceiling-mounted setup may provide an alternative for ORs with especially limited floor space, offering a clear line of sight and proximity to the monitor without obstructing the movement of OR staff or equipment.

In the case of a stand or cart, the mounting device may be suitable for partially extending transversely under the operating table. For instance, a part or all of the stand or the cart may be arrangeable under the operating table. In this way, the mounting device may require no or only little extra space in the OR.

For placing the display above the patient, the mounting device may further comprise an arm suitable for extending next to the operating table. The arm may be arranged at the cart or stand, leading past the side of the operating table and holding the display above it. This may bring the display closer to the surgeon, improving visibility and ergonomic comfort.

Conventional setups may utilize large monitors on sizable carts, which are placed at a distance or off-center of the patient. The large carts may take up valuable space in the OR, creating obstacles for staff and limiting room for other essential equipment. Placing the monitor away from the patient's bed may also result in non-optimal, restricted viewing angles, causing surgeons to strain or assume uncomfortable positions to obtain the correct perspective. The large distance to the display may affect the surgeon's depth perception and sense of control during surgery. These conventional monitors and their carts may also be less flexible in their positioning and restricted in their adjustability for height, angle, or tilt, constraining their ergonomic versatility. These deficiencies may hinder surgical efficacy and contribute to fatigue, strain and discomfort for the surgeon during extended procedures.

By contrast, the mounting device proposed herein may be more space-efficient due to significantly less footprint. The display may be placed closer to the surgeon and in a better viewing angle. The proposed setup may additionally offer significant adjustability in both position and angle.

Examples provide a display system, comprising the above mounting device and the display attached to the mounting device. The mounting device comprises a base arranged at the movable cart or the stand at which the arm is arranged and a mount for the display arranged at the arm. For instance, the mount may be arranged at a vertical edge of the arm. There is a free space between the movable cart or the stand and the display. The arm is arranged at one side of the display system and there is the free space on the other side. The free space can then be used for placement of an operating table. For instance, the display attached to the mount, the arm and the base may jointly form a C-shape, encompassing the edge of the operating table.

The display may be a 3D display, for example. The display may be for displaying an image captured by the microscope or an image derived thereof. For instance, the display system may comprise an interface configured to receive a display signal based on an image captured by the microscope and send the display signal to the display.

The base of the mounting device may refer to the foundational component or structure that provides stability and support for the entire display system, e.g., a weighted or wide platform at the bottom of the cart or the stand for balancing the weight of the display and the rest of the display system.

For instance, the arm or a part thereof may be arranged at a horizontal edge of the base or at least arranged offset to one side of the base rather than being centrally positioned or aligned, i.e., the arm may be positioned more to the edge than to the midpoint of the base. The arm may extend vertically from the side of the base rather than from its center. This may correspond to an asymmetrical arrangement of the arm on the base. For example, the arm may have a central axis along which it extends; and the central axis may be offset in a second different direction, e.g., horizontally, by more than 30 centimeters from a (geometric) center point or center of gravity of the base.

The arm may extend from the base into a first direction. The extension of the arm may be partially vertical or have at least a major vertical portion along the arm. **In** this way, the arm may pass next to the operating table.

The display system may provide a standing or rollable mount for the display. It may be easily moved to different positions in an operating room. The C-shaped design may save space since the main part of the base may be placed under the operating table while the display may be placed close to the surgeon above the patient.

Optionally, the display system may provide robotic or automated adjustment (positioning) mechanisms, ensuring the monitor is aligned with a certain surgeon's viewing position. For such cases, the display system may comprise at least one input interface (e.g., interface circuitry) configured to receive data indicating a target position or movement direction of the display relative to (a position or orientation of) a microscope or relative to (a position or orientation of) a user of the microscope, an actuator and control circuitry configured to control the actuator to move at least a display mounting structure of the mounting device for adjusting a position of the display based on the data.

The display mounting structure may be, for example, the mounting device itself (e.g., in case it is fully movable by wheels, rails or alike) or a part thereof (e.g., a joint, an arm such as a telescope or articulating arm, tilt-and-swivel mechanisms, pivot or gimbal mounts).

For instance, the interface may be configured to establish a communicative connection with an external or integrated device capable of determining the target position or movement direction. This device may include a user interface that enables the user to input the target position or movement direction. Additionally, or as an alternative, the target position or movement direction may be determined by sensors. One or more sensors may be integrated within the display system and may be utilized to determine the target position or movement direction.

The term target position may refer to a specific spatial location that the display mounting structure is intended to reach or align with. This position may be defined, e.g., in terms of coordinates within a predefined space or frame of reference. These coordinates may be used to direct the actuator to align the display mounting structure with the microscope or the user. It may serve as a spatial goal for the actuator's operation. The term movement direction may refer to the trajectory or path along which the display mounting structure is intended to travel or adjust itself to transition toward a target position. It may be defined as a vector or an angular orientation within the display system's operational space. The data may specify the direction and, in some cases, the magnitude of movement. Generally, the data may comprise an indication of how the actuator has to be controlled or of how the display mounting structure has to be moved in order to reach a desired or target position relative to the microscope or user. The needed parameters could be determined or provided through user input, automated computations within the system, or sensor-derived data to guide the system's operation.

The actuator is a mechanical device, e.g., a stepper or servo motor, that executes the physical movements or adjustments needed to align the display with the specified position. For instance, the arm may comprise the actuator for fine-tuned adjustments in at least one or all degrees of freedom (e.g., vertical, horizontal, rotational). For example, the actuator may be configured to move the mounting device or the part thereof for adjusting at least one of a horizontal position of the display, a vertical position of the display, an angular orientation of the display or a relative distance of the display to the microscope or the user.

Optionally, obstacle avoidance may be provided. Built-in sensors may detect obstacles (e.g., other equipment or staff) and adjust the monitor's path to avoid collisions. For example, the display system may comprise at least one optical sensor (e.g., lidar, radar, proximity sensors) configured to detect objects in an environment of the mounting device or the display (e.g., in a specified distance to the mounting device or the display). The display system may comprise processing circuitry configured to determine a potential collision of the mounting device or the display with the detected objects. For instance, the processing circuitry may use techniques like image processing or signal analysis to identify the objects and determine their properties like position, speed and direction of movement, or shape and size (e.g., a bounding box or contour). Then, the processing circuitry may determine a potential collision, e.g., based on bounding box intersections, proximity thresholds or kinematic equations.

The control circuitry may be configured to control the actuator to move the display mounting structure based on the determined potential collision, e.g., such that the collision is avoided. For instance, the control signal for the actuator may be adjusted accordingly.

To ensure precise alignment, the display system may optionally incorporate feedback mechanisms. For example, the display system may comprise a gyroscope or an accelerometer configured to measure a movement of the display system or a part thereof. The control circuitry is configured to control the actuator to move the display mounting structure based on the measured movement. This may enable real-time feedback, e.g., using positional sensors on the monitor arm to continuously verify its position relative to the surgeon. Errors in the position, such as minor deviations caused by accidental bumps or vibrations in the OR, may be automatically corrected.

In some examples, the display system comprises an optical sensor configured to generate sensor data indicating a position of the microscope or a position of the user of the microscope. The control circuitry is configured to control the actuator to move the display mounting structure based on the sensor data. For instance, the sensor data or data derived thereof may be used to move the display mounting structure to the target position or along the movement direction.

The optical sensor may be any device capable of detecting and measuring light or variations in light patterns to generate sensor data about the surrounding environment. For example, it may be a camera module detecting the user's head position or measuring the position of the microscope within the operating room. The sensor may generate sensor data, e.g., information indicative of specific positional or movement details. This may include distance and orientation of the microscope or data about the user's head position or eyes position or gaze direction relative to the microscope, display or relative to a further object between the user and the display. The further object may be the patient or a body part of the patient to be operated on.

As an alternative, body-position sensors may be used. That is, wearable sensors on the surgeon (e.g., on their scrubs or headband) may communicate with the display system to determine the surgeon's posture and adjust the monitor position.

The display system may, for instance, provide automated alignment through, e.g., eye-tracking with sensors integrated into the display or other parts of the display system. In this way, the monitor's position may be aligned to match the line of sight. For example, a camera on the monitor or arm identifies the surgeon's face and tracks their head movements. The monitor position may be adjusted, e.g., in real time, to maintain optimal alignment. Facial recognition software may be used to detect key facial landmarks (e.g., eyes, nose, head orientation). The surgeon's head movements may be tracked, and the monitor may be adjusted accordingly in real time to maintain alignment. For a dynamic field of view, the display system may ensure that the monitor is positioned to maximize the surgeon's depth perception and comfort. A fail-safe mechanism may be integrated: If the display system loses track of the surgeon's face (e.g., due to obstruction), it may default to the last known position or a preset configuration.

In some examples, the interface is configured to receive further data indicating a user-defined position of the display. The control circuitry is configured to control the actuator to move the display mounting structure based on the further data. For instance, the user might input this data through a touchscreen, a keyboard, a joystick, or other input devices, specifying a desired tilt, rotation, location or alike for the display. The control circuitry may process the user-defined position data received through the interface and interpret the input data, determine the adjustments required, and generate control signals for the actuator to achieve the user-defined display position.

The user input may be used as override: Users may in this way manually override an automated adjustment, e.g., via a control panel, touchscreen, or joystick. Optionally, the user may physically adjust the monitor for fine control.

Optionally, preset configurations may be provided. That is, users can define and save individual preferred monitor positions, e.g., for specific surgical workflows. The display system can recall these presets, e.g., at the touch of a button or automatically depending on the workflow step, decreasing setup time. For example, the display system may comprise memory configured to save a plurality of target positions, and the interface may be configured to receive further data indicating a selection of one of the plurality of target positions. The target positions may be customized for height, distance, angle, and tilt. Presets can be defined for specific procedures (e.g., cataract surgery or retinal surgery) or for specific users. Surgeons or their assistants can activate presets with a user interface, e.g., with a button on the cart, microscope interface, or using a footswitch, which simplifies the use of the adjustment possibilities. An application example may be a first preset with a close monitor positioning for high magnification cataract surgery with an upright angle and a second preset with slightly distant positioning with a tilted angle for wide-field retinal visualization.

**In** case of automated selection of the target positions, the interface may be configured to receive further data indicating a data connection to an external device (e.g., an intraoperative device). The display system may comprise processing circuitry configured to select one of the plurality of target positions based on the further data, e.g., based on which type of external device is connected. The display system may thus provide advanced algorithms for workflow recognition. Surgical workflow stages may be automatically detected for adjusting the monitor accordingly. The display system may connect with an intraoperative device (e.g., phacoemulsification or vitrectomy machines) to derive the surgical step. The display position adjustments may be workflow-specific, i.e., monitor placement may be adjusted individually for different stages of surgery (e.g., the position may be closer for cataract incisions, further away for posterior capsular procedures). Surgeons may still be able to set specific adjustments for each workflow stage.

In some examples, the display system may further comprise a microphone configured to capture a sound in an environment of the display system and processing circuitry configured to detect a voice command of the user of the microscope based on the captured sound. The control circuitry is configured to control the actuator based on the detected voice command. For instance, the microphone may capture audio signals including spoken words or commands issued by the user. The user might utter a voice command such as "tilt up" or "move monitor closer" which the microphone records for further processing.

The processing circuitry may employ techniques like noise reduction and filtering to isolate the relevant audio signals. Algorithms, such as speech recognition or natural language processing, may be applied to detect and interpret the user's voice commands. These commands are then translated into actionable instructions.

This system enables hands-free operation, enhancing usability and efficiency, particularly operating rooms requiring minimal manual interaction. Voice-activated commands may minimize disruptions during surgery. Surgeons can use predefined voice commands for instant adjustments.

In some examples, the interface is further configured to receive second data indicating a target position or movement direction of a second display relative to the microscope or relative to the user of the microscope. The display system may further comprise a second actuator. The control circuitry is further configured to control the second actuator to move at least a second display mounting structure of the mounting device for adjusting a position of the second display based on the second data. This dual-monitor setup may be implemented for specific procedures combining, e.g., a smaller overhead monitor with a larger monitor. This may allow the surgeon to choose the optimal display based on procedure phase or specific preferences.

Examples provide a movable cart for a display. The movable cart comprises at least one input interface configured to receive data indicating a target position or movement direction of the display relative to a microscope or relative to a user of the microscope. The movable cart further comprises an actuator and control circuitry configured to control the actuator to move the movable cart for adjusting a position of the display based on the data. The movable cart and its automatic positioning abilities may improve the flexible placement of the monitor which reduces interruptions to the OR workflow.

Examples provide a mounting device for a display. The mounting device comprises a base arranged at a movable cart or a stand and an arm arranged at the base and extending in a first direction, e.g., substantially vertical. A central axis of the arm is offset in a second direction, e.g., substantially horizontal, by more than 30 centimeters from a center point of the base. The mounting device further comprises a mount for the display arranged at the arm. The mounting device may have the configuration of the mounting device described above. For instance, the arm may be arranged at a horizontal edge of the base, and the mount may be arranged at a vertical edge of the arm. A distance between the mount and the base may be more than 100 centimeters.

The mounting device may, in some examples, comprise at least one input interface configured to receive data indicating a target position or movement direction of the display relative to a microscope or relative to a user of the microscope, an actuator and control circuitry configured to control the actuator to move a display mounting structure of the mounting device for adjusting a position of the display based on the data.

Examples provide an optical imaging system, comprising the display system described above and the microscope. The display system is configured to display an image captured by the microscope or an image derived thereof. In some examples, the display system and the microscope are spatially separated.

In some examples, the microscope and the display system are communicatively coupled. The microscope comprises an optical sensor configured to detect a position of the display or a position of the user of the microscope. The control circuitry is configured to control the actuator to move the display mounting structure based on the detected position.

This may enable a sensor-based alignment: A sensor mounted on the microscope, or its optics carrier may track the surgeon's position and adjust the monitor automatically. This may ensure the monitor remains aligned with the surgeon's line of sight, even during movement. A positional sensor (e.g., infrared or ultrasonic) may be used mounted on the microscope's optics carrier or an accessory (e.g., camera or handles). The sensor may gather data on the surgeon's head position or eye level in relation to the microscope and patient bed. The monitor arm may use this data to adjust height, distance, angle, and tilt automatically and in real-time to ensure the surgeon's optimal line of sight.

Examples provide an operating system, comprising the display system described above and the operating table. The display is arranged above the operating table.

Examples provide a method for setting up an operating room, comprising automatically positioning a stand or movable cart of a display mounting device relative to an operating table such that a display can be arranged above the operation table using the display mounting device.

Examples provide a method, comprising receiving data indicating a target position or movement direction of a display relative to a microscope or relative to a user of the microscope and controlling an actuator to move a display mounting structure of the display for adjusting a position of the display based on the data.

Examples provide a computer program with a program code for performing one of the methods above when the computer program is executed on a processor.

Examples further relate to a computer program having a program code for performing the method described above, when the computer program is executed on a computer, a processor, or a programmable hardware component.

### Short description of the Figures

Some examples of apparatuses and/or methods will be described in the following by way of example only, and with reference to the accompanying figures, in which
Fig. 1a and Fig. 1b show schematic illustrations of examples of a mounting device;
Figs. 2a-c show schematic illustrations of examples of an operation scenario with changing positions of the display;
Fig. 3a and Fig. 3b show a technical drawing of an oblique view and a side view of an example of a movable cart;
Fig. 4 shows a schematic illustration of an example of a mounting device;
Fig. 5 shows a schematic illustration of an example of an optical imaging system;
Fig. 6 shows a schematic illustration of an example of an operating system;
Fig. 7 shows a schematic illustration of an example of a method for setting up an operating room;
Fig. 8 shows a schematic illustration of an example of a method;
Fig. 9 shows a schematic illustration of an example of a system; and
Fig. 10 shows a schematic illustration of an example of an optical imaging system.

### Detailed Description

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Various examples will now be described more fully with reference to the accompanying drawings in which some examples are illustrated. In the figures, the thicknesses of lines, layers and/or regions may be exaggerated for clarity.

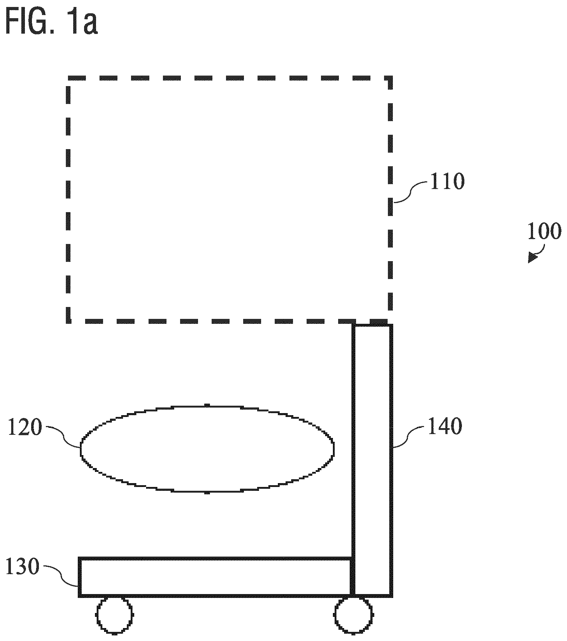

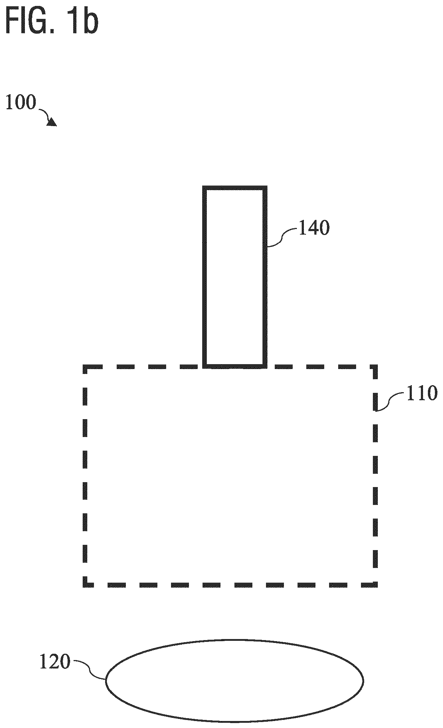

Fig. 1a and Fig. 1b show schematic illustrations of examples of a mounting device 100. The mounting device 100 is suitable for arranging a display 110 above an operating table 120.

In Fig. 1a, the mounting device 100 comprises a movable cart 130 suitable for extending transversely under the operating table 120 and an arm 140 suitable for extending next to the operating table 120.

The movable cart 130 comprises a flat platform (base) with wheels. The arm 140 is positioned at a horizontal edge of the platform and extends vertically upwards, passing alongside the operating table 120. An edge of the display 110 is positioned at the vertical end of the arm 140, allowing the main portion of its horizontal span to remain unobstructed, creating free space beneath it to accommodate the placement of the operating table 120.

In the example of Fig. 1a, the display 110, the arm 140, and the platform of the movable cart 130 are structurally arranged to form a C-shaped configuration. Specifically, the platform serves as the base of the "C" providing stability and housing the components needed for mobility. The arm 140 extends vertically from one end of the platform, representing the spine of the "C" while the display 110 is mounted at the vertical end of the arm, forming the top segment of the "C". This configuration provides open space within the interior of the "C" allowing the operating table 120 or other equipment to be positioned within this area without obstruction. The arrangement may ensure optimal accessibility to the display and facilitate ergonomic operation and efficient use of workspace, particularly in clinical or laboratory environments.

Generally, a mounting device 100 as proposed herein may be described as follows: The arm 140 allows an arrangement of the display 110 such that there is a free space between the movable cart 130 and the display 110. The arm 140 is arranged at one side of the mounting device 100, and the free space is on the other side.

As an alternative to the example of Fig. 1a, the arm 140 or a part thereof may be arranged not at the very edge of the platform but offset to one side of the platform rather than being centrally positioned or aligned, i.e., the arm 140 may be positioned more to the edge than to the midpoint of the platform. For example, the arm 140 may have a central axis along which it extends; and the central axis may be offset, e.g., horizontally, by more than 30 centimeters from a (geometric) center point or center of gravity of the platform.

Other than shown in Fig. 1a, the arm 140 may extend from the platform generally into a first direction. The extension of the arm 140 may be at least partially vertical or have at least a major vertical portion along the arm 140 such that it can extend next to the operating table 120.

The C-shaped design may save space since the main part of the movable cart 130 may be placed under the operating table 120 while the display 110 may be placed close to the surgeon above the patient.

In Fig. 1b, the arm 140 of the mounting device 100 is arranged above the display, i.e., the mounting device 100 is specifically designed for attachment to a ceiling structure. This ceiling-mounted configuration allows the arm 140 to extend downward, suspending the display 110 over the operating table 120. This design may be particularly advantageous in environments where floor or wall space is limited, as in operating rooms.

As an alternative to the configurations shown in Fig. 1a and Fig. 1b, the mounting device 100 may comprise a wall-mounted arm, a microscope-mounted arm or a stand-mounted arm. For the former, a flexible arm may be attached to the OR wall. For the microscope integration, a flexible arm may be attached to the microscope. For the latter, a flexible arm may be attached to a bedside stand. These alternatives may provide adjustability and space-saving benefits.

Conventionally, a monitor may be placed far away to the side of the patient forcing the surgeon to rotate their head to the side to see the monitor. The space at the feet of the patient may be taken up by the anesthetist and required machines, hence the monitor cannot be placed straight ahead.

By contrast, the mounting device 100 as proposed may comprise, e.g., a flexible arm mechanism attached to a compact cart or ceiling mount. The cart can be positioned below or beside the patient bed, while the ceiling mount may hang the monitor directly over the patient bed. The mounting device 100 may also enable the use of a smaller 3D monitor. The monitor's adjustable features may ensure ergonomic positioning for the surgeon. The monitor's closer proximity may enhance the surgeon's sense of control, as the reduced viewing distance aids depth perception.

More details and aspects of the mounting device 100 are mentioned in connection with the examples described above and/or below. The example shown in Figs. 1a and 1b may comprise one or more optional or additional features corresponding to one or more aspects mentioned in connection with the proposed concept or one or more other examples described herein (e.g., with reference to the summary above).

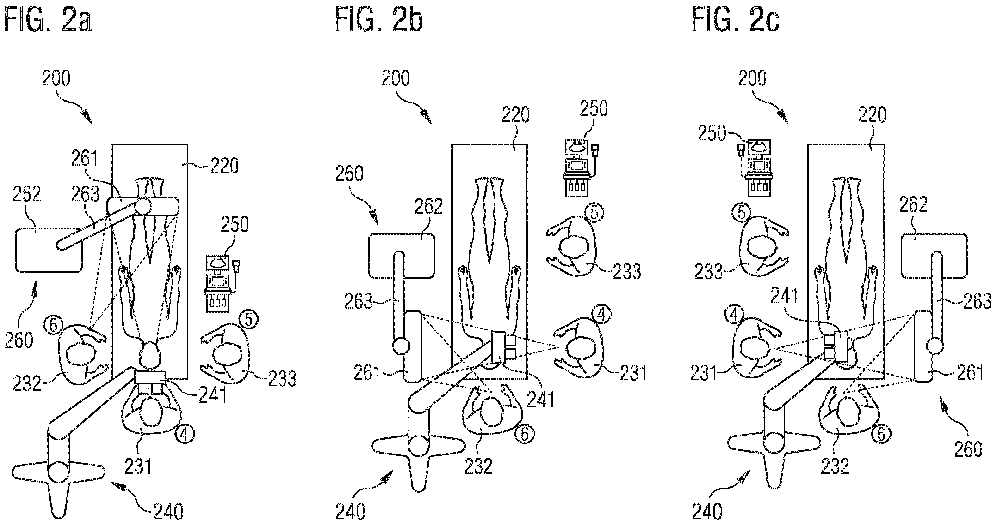

Figs. 2a-c provide schematic representations of an example operation scenario 200, such as during a brain surgery, featuring an example of a display system 210 as described herein. The illustrations depict the display system 210 positioned in various locations within the operation scenario 200 to demonstrate its adaptability and functionality.

The operation scenario 200 illustrates an operating table 220 positioned at the center, with three medical staff members 231, 232, and 233, such as a lead surgeon, an assistant surgeon, and a sterile nurse or similar personnel, arranged around the table and oriented toward the patient.

One of the medical staff members 231, for example, the lead or main surgeon, uses the microscope 241 and when looking up has a display 261 aligned with their view for examination. The display 261 may show an image captured by the microscope 241 or an image derived thereof. Throughout this application, this individual 231 is referred to as the "user" of the microscope 241. In the example illustrated in Fig. 2a, the user 231 is positioned at the head of the operating table 220, while the other medical staff members 232 and 233 stand near the patient's head on the left and right sides of the operating table 220, respectively. In the examples depicted in Figs. 2b and 2c, the user 231 is positioned at the right and left sides of the operating table 220, respectively. In all these scenarios, as shown in Figs. 2a-c, the microscope 241 is arranged in front of the user 231 to provide access to the microscope 241 for the user 231.

In the examples depicted in Figs. 2a-c, the microscope 241 is equipped with oculars that allow the user 231 to view the patient directly. However, the use of these oculars is optional when the display 261 presents the image captured by the microscope. In other variations, the microscope may lack oculars entirely, and the user 231 may rely exclusively on the display 261 to view the microscopic image.

In the examples shown in Figs. 2a-c, the microscope 241 is a component of a microscope system 240, which includes a stand equipped with an adjustable arm. This arm allows the microscope 241 to be swiveled into various positions, such as toward the head end of the operating table 220, as depicted in Fig. 2a, or towards the sides of the table, as illustrated in Figs. 2b and 2c.

A phaco-vitrectomy machine 250 is positioned adjacent to staff member 233 and is relocated as needed to correspond with the changes in the position of staff member 233 shown in Figs. 2a-c.

As the position of the user 231 and the orientation of the microscope 241 change across Figs. 2a-c, the viewing angle shifts accordingly. It transitions from being directed toward the foot end of the operating table 220 in Fig. 2a to being oriented to the left and right sides in Figs. 2b and 2c, respectively.

The operation scenario 200 includes a mounting device 260 that holds the display 261. The mounting device 260 is designed to position the display 261 above the operating table 220, as described herein and depicted in Fig. 2a. In the example shown in Figs. 2a-c, this is achieved using a stand or rollable base 262 equipped with an arm 263 that extends alongside the operating table 220. The arm 263 supports the display 261, enabling flexible positioning above or beside the operating table 220 with various orientations. This may ensure that the display 261 remains aligned with the user's 231 shifting viewing angles. To transition from the left side of the operating table 220, as shown in Fig. 2b, to the right side in Fig. 2c, the mounting device 260 can be relocated, for example, by utilizing wheels on the base 262.

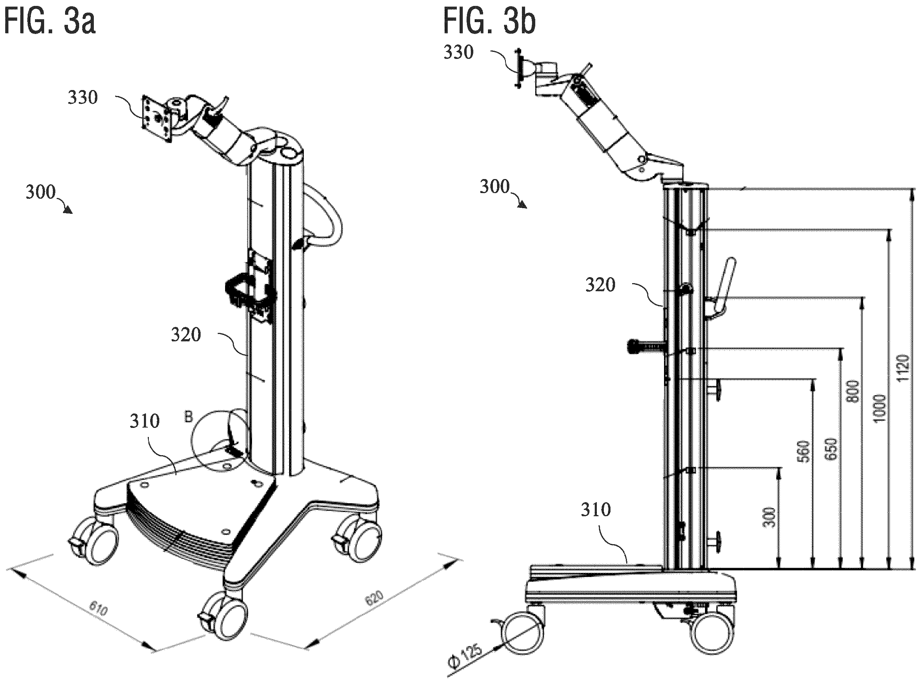

Fig. 3a and Fig. 3b illustrate a side view and an oblique view of an example of a mounting device 300 suitable for arranging a display above an operating table. The mounting device 300 includes a base 310 and an arm 320. The base 310 is designed to extend transversely beneath an operating table, with a portion of the base 310 projecting forward. Meanwhile, the arm 320 is positioned offset toward the rear of the base, making it suitable for extending alongside the operating table.

The base 310 is equipped with three wheels or casters for mobility. The base width and length are approximately 610 mm and 620 mm. The protruding part of the base 310 is a forward-extending section. This portion projects outward, providing additional reach and support while ensuring the base 310 can partially slide beneath an operating table. It extends forward beyond the arm's 320 mounting point. This may allow the base 310 to remain stable while positioning the arm 320 further away from the center of the base 310.

The arm 320 has a long, vertical pole that provides an adjustable mounting system for a display. The arm 320 is segmented with adjustment mechanisms, such as joints and pivots, enabling movement in various directions (e.g., swiveling or tilting). Fig. 3a and Fig. 3b depict marked measurements as examples of dimensions of the mounting device 300. The arm 320 has lengths labeled at 300 mm (millimeters), 560 mm, 800 mm, 1000 mm, and 1120 mm, which correspond to extendable or adjustable segments. The arm 320 includes grooves and channels that may be used for routing cables.

At the end of the arm 320, a mounting bracket 330 is shown, which would hold the display. The bracket 330 may allow for tilting and angular adjustments of the attached display. The arm 320 includes a bent portion at its distal end. This portion is a curved, angled segment. The curvature or angular bend may allow the display mounting bracket 330 to be oriented at various angles. A free space is formed by the bent portion of the arm 320 protruding forward and the projecting portion of the base 310. This free space can be used for the placement of the operating table.

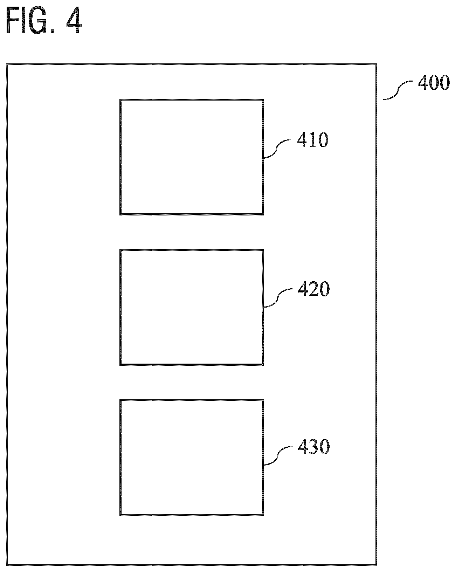

Optionally, a mounting device or a display system as described herein may have automatic positioning capabilities, as described with reference to Fig. 4:
Fig. 4 illustrates a schematic representation of an example of a mounting device 400 (or a display system). The mounting device 400 comprises at least one input interface 410 configured to receive data indicating a target position or movement direction of a display to be mounted on the mounting device 400 relative to a microscope or relative to a user of the microscope. The mounting device 400 further comprises an actuator 420 and control circuitry 430 configured to control the actuator 420 to move at least a display mounting structure of the mounting device for adjusting a position of the display based on the data.

During procedures, the adjustable features of the mounting device 400 may enable surgeons to configure the display to reduce fatigue and maintain posture, regardless of their position relative to the patient. For instance, during lengthy surgeries, surgeons can easily adjust the monitor viewing angle to match their working position, possibly without the need to reposition the mounting device 400.

More details and aspects of the mounting device 400 (or display system) are mentioned in connection with the examples described above and/or below. The example shown in Fig. 4 may comprise one or more optional or additional features corresponding to one or more aspects mentioned in connection with the proposed concept or one or more other examples described herein (e.g., with reference to the summary above).

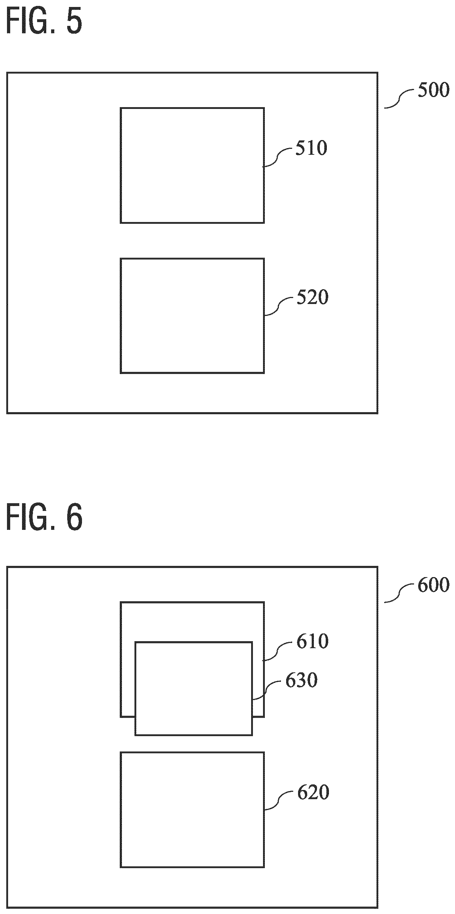

Fig. 5 illustrates a schematic representation of an example of an optical imaging system 500. The optical imaging system 500 comprises a display system 510 as described herein (such as comprising a mounting device as described herein and a display) and a microscope 520. The display system 510 is configured to display an image captured by the microscope 520 or an image derived thereof.

Fig. 6 illustrates a schematic representation of an example of an operating system 600. The operating system 600 comprises a display system 610 as described herein and an operating table 620. The display 630 is arranged above the operating table 620.

Mounting devices and display systems as described herein may enable the usage of a smaller display (e.g., smaller than conventional 55-inch models), requiring less space in the OR. Optional 3D display capability may enhance depth perception, making it particularly valuable for heads-up surgeries. The mounting devices may offer full adjustability, including height, angle, and tilt modifications, to ensure surgeon comfort. The flexible placement of the display, whether on a cart or ceiling-mounted, may minimize disruptions to the OR workflow by reducing the need to reposition a large, heavy cart. Additionally, an automated alignment and robotic adjustment system may ensure the monitor is aligned with the surgeon's viewing position.

The workflow integration is designed to optimize efficiency at every stage. During pre-surgery setup, the monitor is positioned over the patient bed and adjusted for optimal viewing. Throughout the surgery, surgeons can make quick adjustments to the monitor to maintain an ergonomic posture, regardless of their position relative to the patient, reducing strain and fatigue. After the procedure, the monitor and cart can be easily repositioned or transported to another OR.

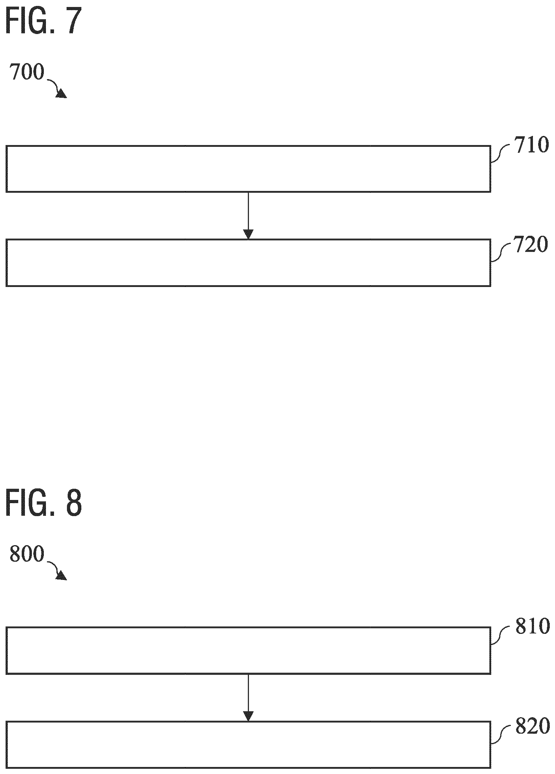

Fig. 7 illustrates a schematic representation of an example of a (e.g., computer-implemented) method 700 for setting up an OR. The method 700 comprises automatically positioning 710 a stand or movable cart of a display mounting device relative to an operating table such that a display can be arranged 720 above the operation table using the display mounting device.

Fig. 8 illustrates a schematic representation of an example of a (e.g., computer-implemented) method 800 comprising receiving 810 data indicating a target position or movement direction of a display relative to a microscope or relative to a user of the microscope and controlling 820 an actuator to move a display mounting structure of the display for adjusting a position of the display based on the data.

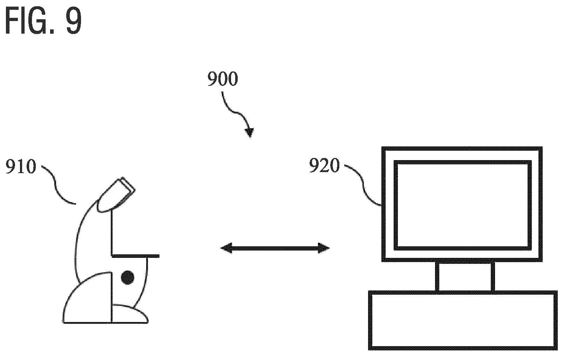

Some embodiments relate to a microscope system comprising an apparatus as described in connection with one or more of the Figs. 1a, 1b, 2a-c, 3a, 3b, 4, 5 or 6. Alternatively, a microscope system may be part of or connected to an apparatus as described in connection with one or more of the Figs. 1a, 1b, 2a-c, 3a, 3b, 4, 5 or 6. Fig. 9 shows a schematic illustration of a system 900, e.g., an optical imaging system, configured to perform a method described herein, e.g., with reference to Figs. 7 or 8. The system 900 comprises a microscope 910 and a computer system 920. The microscope 910 is configured to take images and is connected to the computer system 920. The computer system 920 is configured to execute at least a part of a method described herein. The computer system 920 may be configured to execute a machine learning algorithm. The computer system 920 and microscope 910 may be separate entities but can also be integrated together in one common housing. The computer system 920 may be part of a central processing system of the microscope 910 and/or the computer system 920 may be part of a subcomponent of the microscope 910, such as a sensor, an actor, a camera or an illumination unit, etc. of the microscope 910.

The computer system 920 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 920 may comprise any circuit or combination of circuits. In one embodiment, the computer system 920 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 920 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 920 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 920 may also include a display device, one or more speakers, and a keyboard and/or controller, which can include a mouse, trackball, touch screen, voice-recognition device, or any other device that permits a system user to input information into and receive information from the computer system 920.

More details and aspects are mentioned in connection with the examples described above. The example shown in Fig. 9 may comprise one or more optional or additional features corresponding to one or more aspects mentioned in connection with the proposed concept or one or more examples described above.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

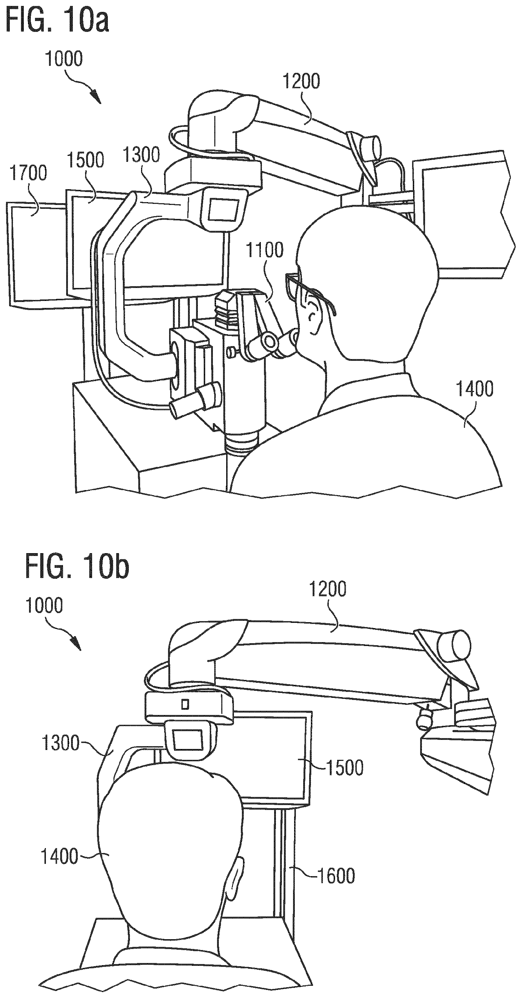

Fig. 10a and Fig. 10b illustrate an oblique view and a back view of an example of an optical imaging system 1000. The optical imaging system 1000 includes a microscope system that comprises a microscope 1100 and a microscope arm 1200. The arm features a C-shaped distal end 1300 that supports the microscope 1100. This C-shaped design of the microscope arm 1200 may allow a user 1400 to maintain an unobstructed view in front when lifting their gaze from the microscope 1100. The use of the oculars of the microscope 1100 is optional since the microscopic image is shown on a display 1500. The imaging system 1100 may be configurable to remove the oculars, then the surgeon may only use the monitor 1500. In other examples than the one shown in Fig. 10a and Fig. 10b, the microscope system does not comprise any oculars.

The optical imaging system 1000 further includes a display system as described herein with the display 1500 and a mounting device 1600 for the display 1500. The mounting device 1600 has an arm positioned at the side of the display 1500, allowing free space beneath the display 1500 and enabling it to be positioned above an operating table.

For comparison, a conventional display 1700 is illustrated in Fig. 10a. The conventional display 1700 is positioned farther from the user 1400 and is larger in size to achieve the same image resolution as the display 1500.

In the following, some examples of the proposed concept are presented:
An example (e.g., example 1) relates to a mounting device suitable for arranging a display above an operating table.

Another example (e.g., example 2) relates to a previous example (e.g., example 1) or to any other example, further comprising a movable cart or a stand suitable for extending transversely under the operating table, and an arm suitable for extending next to the operating table.

An example (e.g., example 3) relates to a display system, comprising the mounting device of example 2, and the display attached to the mounting device, wherein the mounting device comprises a base arranged at the movable cart or the stand, wherein the arm is arranged at the base, and a mount for the display arranged at the arm, wherein there is a free space between the movable cart or the stand and the display, wherein the arm is arranged at one side of the display system, and wherein there is the free space on the other side.

Another example (e.g., example 4) relates to a previous example (e.g., example 3) or to any other example, further comprising at least one input interface configured to receive data indicating a target position or movement direction of the display relative to a microscope or relative to a user of the microscope, an actuator, and control circuitry configured to control the actuator to move at least a display mounting structure of the mounting device for adjusting a position of the display based on the data.

Another example (e.g., example 5) relates to a previous example (e.g., one of the examples 3 or 4) or to any other example, further comprising an optical sensor configured to generate sensor data indicating a position of the microscope or a position of the user of the microscope, wherein the control circuitry is configured to control the actuator to move the display mounting structure based on the sensor data.

Another example (e.g., example 6) relates to a previous example (e.g., example 5) or to any other example, further comprising that the optical sensor is configured to detect a position of the head or eyes of the user relative to the microscope or relative to a further object between the user and the display.

Another example (e.g., example 7) relates to a previous example (e.g., one of the examples 4 to 6) or to any other example, further comprising that the interface is configured to receive further data indicating a user-defined position of the display, wherein the control circuitry is configured to control the actuator to move the display mounting structure based on the further data.

Another example (e.g., example 8) relates to a previous example (e.g., one of the examples 4 to 7) or to any other example, further comprising memory configured to save a plurality of target positions, wherein the interface is further configured to receive further data indicating a selection of one of the plurality of target positions.

Another example (e.g., example 9) relates to a previous example (e.g., one of the examples 4 to 8) or to any other example, further comprising memory configured to save a plurality of target positions, wherein the interface is configured to receive further data indicating a data connection to an external device, and wherein the display system comprises processing circuitry configured to select one of the plurality of target positions based on the further data.

Another example (e.g., example 10) relates to a previous example (e.g., one of the examples 4 to 9) or to any other example, further comprising that the control circuitry is configured to control the actuator to move the movable cart, the arm or the mount for adjusting the position of the display.

Another example (e.g., example 11) relates to a previous example (e.g., one of the examples 4 to 10) or to any other example, further comprising that the actuator is configured to move the display mounting structure for adjusting at least one of a horizontal position of the display, a vertical position of the display, an angular orientation of the display or a relative distance of the display to the microscope or the user.

Another example (e.g., example 12) relates to a previous example (e.g., one of the examples 4 to 11) or to any other example, further comprising at least one optical sensor configured to detect objects in an environment of the mounting device or the display, and processing circuitry configured to determine a potential collision of the mounting device or the display with the detected objects, wherein the control circuitry is configured to control the actuator to move the display mounting structure based on the determined potential collision.

Another example (e.g., example 13) relates to a previous example (e.g., one of the examples 4 to 12) or to any other example, further comprising a gyroscope or an accelerometer configured to measure a movement of the display system or a part thereof, wherein the control circuitry is configured to control the actuator to move the display mounting structure based on the measured movement.

Another example (e.g., example 14) relates to a previous example (e.g., one of the examples 4 to 13) or to any other example, further comprising a microphone configured to capture a sound in an environment of the display system, and processing circuitry configured to detect a voice command of the user of the microscope based on the captured sound, wherein the control circuitry is configured to control the actuator based on the detected voice command.

Another example (e.g., example 15) relates to a previous example (e.g., one of the examples 4 to 14) or to any other example, further comprising that the interface is further configured to receive second data indicating a target position or movement direction of a second display relative to the microscope or relative to the user of the microscope, and wherein the display system further comprises a second actuator, wherein the control circuitry is further configured to control the second actuator to move at least a second display mounting structure of the mounting device for adjusting a position of the second display based on the second data.

Another example (e.g., example 16) relates to a previous example (e.g., one of the examples 3 to 15) or to any other example, further comprising that the display attached to the mount, the arm and the base jointly form a C-shape.

Another example (e.g., example 17) relates to a previous example (e.g., one of the examples 3 to 16) or to any other example, further comprising that the interface is configured to receive a display signal based on an image captured by the microscope and send the display signal to the display.

Another example (e.g., example 18) relates to a previous example (e.g., one of the examples 3 to 17) or to any other example, further comprising that the display is a 3D display.

An example (e.g., example 19) relates to a movable cart for a display, comprising at least one input interface configured to receive data indicating a target position or movement direction of the display relative to a microscope or relative to a user of the microscope, an actuator, and control circuitry configured to control the actuator to move the movable cart for adjusting a position of the display based on the data.

An example (e.g., example 20) relates to a mounting device for a display, comprising a base arranged at a movable cart or a stand, an arm arranged at the base and extending in a first direction, wherein a central axis of the arm is offset in a second direction by more than 30 centimeters from a center point of the base, and a mount for the display arranged at the arm.

Another example (e.g., example 21) relates to a previous example (e.g., example 20) or to any other example, further comprising that the arm is arranged at a horizontal edge of the base, and wherein the mount is arranged at a vertical edge of the arm.

Another example (e.g., example 22) relates to a previous example (e.g., one of the examples 20 or 21) or to any other example, further comprising at least one input interface configured to receive data indicating a target position or movement direction of the display relative to a microscope or relative to a user of the microscope, an actuator, and control circuitry configured to control the actuator to move a display mounting structure of the mounting device for adjusting a position of the display based on the data.

Another example (e.g., example 23) relates to a previous example (e.g., one of the examples 20 to 22) or to any other example, further comprising that a distance between the mount and the base is more than 100 centimeters.

An example (e.g., example 24) relates to an optical imaging system, comprising the display system of any one of examples 3 to 18, and the microscope, wherein the display system is configured to display an image captured by the microscope or an image derived thereof.

Another example (e.g., example 25) relates to a previous example (e.g., example 24) or to any other example, further comprising that the display system and the microscope are spatially separated.

Another example (e.g., example 26) relates to a previous example (e.g., one of the examples 24 or 25) or to any other example, further comprising that the microscope and the display system are communicatively coupled, wherein the microscope comprises an optical sensor configured to detect a position of the display or a position of the user of the microscope, and wherein the control circuitry is configured to control the actuator to move the display mounting structure based on the detected position.

An example (e.g., example 27) relates to an operating system, comprising the display system of any one of examples 3 to 18, and the operating table, wherein the display is arranged above the operating table.

An example (e.g., example 28) relates to a method for setting up an operating room, comprising automatically positioning a stand or movable cart of a display mounting device relative to an operating table such that a display can be arranged above the operation table using the display mounting device.

An example (e.g., example 29) relates to a method, comprising receiving data indicating a target position or movement direction of a display relative to a microscope or relative to a user of the microscope, and controlling an actuator to move a display mounting structure of the display for adjusting a position of the display based on the data.

Another example (e.g., example 30) relates to a computer program with a program code for performing the method according to any one of examples 28 or 29 when the computer program is executed on a processor.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 mounting device
110 display
120 operating table
130 movable cart
140 arm
200 operation scenario
210 display system
220 operating table
231, 232, 233 medical staff members
240 microscope system
250 phaco-vitrectomy machine
260 mounting device
261 display
262 base
263 arm
300 mounting device
310 base
320 arm
330 bracket
400 mounting device
410 interface
420 actuator
430 control circuitry
500 optical imaging system
510 display system
520 microscope
600 operating system
610 display system
620 operating table
700 method
710 positioning
720 arranging
800 method
810 receiving
820 controlling
900 system
910 microscope
920 computer system
1000 optical imaging system
1100 microscope
1200 microscope arm
1300 C-shaped segment
1400 user
1500 display
1600 mounting device
1700 conventional display

## Claims

1. A mounting device (100) suitable for arranging a display (110) above an operating table (120).

2. The mounting device of (100) claim 1, further comprising:
a movable cart (130) or a stand suitable for extending transversely under the operating table (120); and
an arm (140) suitable for extending next to the operating table (120).

3. A display system, comprising:
the mounting device (100) of claim 2; and
the display (110) attached to the mounting device (100), wherein the mounting device (100) comprises:
a base (310) arranged at the movable cart (130) or the stand, wherein the arm (320) is arranged at the base (310); and
a mount (330) for the display (110) arranged at the arm (320), wherein there is a free space between the movable cart (130) or the stand and the display (110), wherein the arm (320) is arranged at one side of the display system, and wherein there is the free space on the other side.

4. The display system of claim 3, further comprising:
at least one input interface (410) configured to receive data indicating a target position or movement direction of the display (110) relative to a microscope or relative to a user of the microscope;
an actuator (420); and
control circuitry (430) configured to control the actuator (420) to move at least a display mounting structure of the mounting device (100) for adjusting a position of the display (110) based on the data.

5. The display system of claim 4, further comprising:
memory configured to save a plurality of target positions, wherein the interface (410) is configured to receive further data indicating a data connection to an external device, and wherein the display system comprises processing circuitry configured to select one of the plurality of target positions based on the further data.

6. The display system of any one of claims 3 to 5, wherein
the display (110) attached to the mount (330), the arm (320) and the base (310) jointly form a C-shape.

7. The display system of any one of claims 3 to 6, wherein
the interface (410) is configured to receive a display signal based on an image captured by the microscope and send the display signal to the display (110).

8. A movable cart (400) for a display, comprising:
at least one input interface (410) configured to receive data indicating a target position or
movement direction of the display relative to a microscope or relative to a user of the microscope;
an actuator (420); and
control circuitry (430) configured to control the actuator to move the movable cart for adjusting a position of the display based on the data.

9. A mounting device (300) for a display, comprising:
a base (310) arranged at a movable cart or a stand;
an arm (320) arranged at the base (310) and extending in a first direction, wherein a central axis of the arm (320) is offset in a second direction by more than 30 centimeters from a center point of the base (310); and
a mount (330) for the display arranged at the arm.

10. The mounting device (300) of claim 9, wherein
a distance between the mount (330) and the base (310) is more than 100 centimeters.

11. An optical imaging system (500), comprising:
the display system (510) of any one of claims 3 to 7; and
the microscope (520), wherein the display system (510) is configured to display an image captured by the microscope or an image derived thereof.

12. The optical imaging system (500) of claim 11, wherein
the display system (510) and the microscope (520) are spatially separated.

13. An operating system (600), comprising:
the display system (610) of any one of claims 3 to 7; and
the operating table (620), wherein the display (630) is arranged above the operating table (620).

14. A method (700) for setting up an operating room, comprising:
automatically positioning (710) a stand or movable cart of a display mounting device relative to an operating table such that a display can be arranged (720) above the operation table using the display mounting device.

15. A method (800), comprising:
receiving (810) data indicating a target position or movement direction of a display relative to a microscope or relative to a user of the microscope; and
controlling (820) an actuator to move a display mounting structure of the display for adjusting a position of the display based on the data.
